Europäisches Patentamt

European Patent Office

Office européen des brevets

(19)

(11) Numéro de publication : **0 060 369**
**B1**

(12) **FASCICULE DE BREVET EUROPÉEN**

(45) Date de publication du fascicule du brevet :
**15.08.84**

(21) Numéro de dépôt : **81402088.9**

(22) Date de dépôt : **29.12.81**

(51) Int. Cl.³ : **A 61 M   1/00**

(54) **Valve pour le traitement de l'hydrocéphalie.**

(30) Priorité : **18.03.81 FR 8105389**

(43) Date de publication de la demande :
**22.09.82 Bulletin 82/38**

(45) Mention de la délivrance du brevet :
**15.08.84 Bulletin 84/33**

(84) Etats contractants désignés :
**AT BE CH DE FR GB IT LI NL SE**

(56) Documents cités :
**FR-A- 2 354 103**
**US-A- 3 886 948**

(73) Titulaire : **SOPHYSA, Société dite:**
**Rue des Châtaigniers B.P. 3 Guipel**
**F-35440 Montreuil sur Ille (FR)**

(72) Inventeur : **Marion, Bernard**
**Rue des Châtaigniers B.P. 3 Guipel**
**F-35440 Montreuil sur Ille (FR)**

(74) Mandataire : **Dupuy, René Gaston et al**
**Cabinet René G. Dupuy & Jean M.L. Loyer 14, Rue La Fayette**
**F-75009 Paris (FR)**

Jouve, 18, rue St-Denis, 75001 Paris, France

## Description

La présente invention est relative à une valve pour le traitement de l'hydrocéphalie.

On sait que l'hydrocéphalie est une maladie provoquée notamment par le blocage des sites naturels de résorption du liquide céphalo-rachidien appelés villosités arachnoïdiennes. Ce blocage entraîne une augmentation du volume des ventricules de la cavité crânienne et par conséquent, de l'ensemble du crâne chez les nouveaux-nés dont les fontanelles sont encore ouvertes.

Chez l'adulte, en raison de la rigidité de la boîte crânienne, cette affection se manifeste par des troubles de la démarche, des incontinences, des troubles mentaux et une dégradation progressive du parenchyme cérébral.

Un traitement de cette maladie consiste à dériver le liquide céphalo-rachidien contenu dans les ventricules de la cavité crânienne vers tout autre site de résorption tel que le cœur ou le péritoine.

A cet effet, on introduit un catheter dans l'un des ventricules de la cavité crânienne en pratiquant un trou au trépan dans le crâne du patient, on relie le cathéter à une autre tubulure qui est passée sous le scalp et qui, au niveau du cou, rattrape soit la veine jugulaire, auquel cas on complète l'appareillage en introduisant un cathéter distal dans l'oreillette gauche du cœur, soit simplement le péritoine.

L'ensemble comporte donc un cathéter amont et un cathéter aval mais il constitue en fait un seul système que l'on est obligé de raccorder une ou deux fois selon le modèle. L'ensemble du shunt, de l'extrémité proximale à l'extrémité distale de celui-ci est entièrement sous-cutané ce qui donne au patient une liberté de mouvement et évite les infections.

Les villosités arachnoïdiennes maintiennent une pression constante entre le liquide céphalo-rachidien et le sang veineux. Elles évitent en outre que les ventricules de la cavité crânienne se vident dans la position orthostatique.

Par conséquent, lorsqu'on met en place un système de dérivation du liquide céphalo-rachidien on doit insérer une valve dans ce système.

La valve est placée soit à l'extrémité du système et c'est alors une valve distale, soit près du trou de trépan auquel cas il s'agit d'une valve proximale.

On connaît à l'heure actuelle un certain nombre de types de valves utilisées dans des systèmes de dérivation, mis en place pour le traitement de l'hydrocéphalie.

Parmi ces valves, on peut citer les valves de Pudenz, de Raimondi et de Holter qui sont du type à entailles ménagées dans la paroi du conduit réalisé en un élastomère de silicone, ces entailles s'ouvrant sous l'effet d'une pression déterminée du liquide céphalo-rachidien.

Les valves de Pudenz et de Raimondi comportent des entailles pratiquées à l'extrémité distale, fermée du conduit.

La valve de Holter comporte une enveloppe tubulaire destinée à être disposée à proximité du trou de trépan. Dans les extrémités de cette enveloppe, on introduit des petites cupules en une matière à base de silicones dans lesquelles sont pratiquées des fentes latérales destinées à permettre l'écoulement du liquide céphalorachidien.

On peut également citer la valve de Hakim (US-A-3 288 142 et US-A-3 527 226), qui comporte un dispositif mécanique de contrôle de la pression du liquide. Ce dispositif est constitué par une bille formant clapet, disposée dans un siège tronconique et appliquée contre ce siège par un ressort calibré de façon à permettre l'ouverture de la valve pour une pression déterminée. La valve de Hakim présente sur les valves à entailles, l'avantage de s'ouvrir à une pression précise. Cependant, quelle que soit leur précision de fonctionnement, les valves connues sont prévues pour ne fonctionner que dans une seule plage de pressions, de sorte qu'il est nécessaire de réaliser une gamme de valves prévues pour fonctionner dans différentes plages de pressions adaptées aux divers cas d'hydrocéphalie à traiter.

En outre, il arrive qu'au cours de l'évolution de la maladie ou pendant son traitement, la valve implantée initialement, présente une pression de fermeture trop élevée ou trop basse.

Il est alors nécessaire de remplacer la valve, ce qui implique une nouvelle intervention chirurgicale.

L'invention vise à remédier aux inconvénients que présentent les valves de la technique antérieure, en créant une valve qui tout en présentant une bonne précision de fonctionnement, puisse être réglée de l'extérieur à plusieurs pressions sans que le neuro-chirurgien soit obligé de l'explanter.

On a déjà proposé des valves réglables par exemple celle décrite dans le document FR-A-2 354 103. Cette valve qui est implantée entre le scalp et la boîte crânienne se propose d'effectuer la régulation de la pression du liquide céphalo-rachidien arrivant dans une chambre limitée par une membrane contre laquelle applique un levier actionnant une soupape placée sur le drain d'évacuation. La déformation de la membrane et donc la position du levier est asservie à un palpeur pourvu d'un ressort taré réglable par rotation d'un système à vis/écrou.

Si l'on observe qu'une valve aux fins envisagées doit être essentiellement simple, légère et sûre, on est obligé de conclure que la valve du document FR-A-2 354 103 ne répond pas à ces critères.

Le Dr. Hakim avait proposé dans son brevet US-A-3 886 948 (et FR-A-2 196 176 correspondant) un shunt ventriculaire comportant une valve autoréglable ayant recours à un senseur extra-dural rempli d'un liquide hydraulique radio-opaque. La valve utilisée comportait un clapet à bille soumis à l'action d'un ressort à lame dont l'extrémité libre était fixée sur une palette levier articulée en un point fixe et déplaçable au moyen d'une poche reliée par le liquide hydraulique à celle du senseur placé à l'intérieur de la cavité crânienne au-dessus de la DURE-MERE. Outre que ce dispositif nécessite une longue et délicate opération en raison de la position du senseur, les variations de la pression extra-durale que ce senseur est supposé transmettre au mécanisme de la valve sont tout à fait aléatoires. En effet, ce senseur constitue une chambre hydrostatique déformable, rempli d'huile radio-opaque, placé lui-même dans un milieu déformable (le cortex). Par ailleurs, ce shunt ne donnait pas au neuro-chirurgien la possibilité de modifier, après implantation, la pression de fermeture de la valve.

L'invention se propose de résoudre le problème posé au moyen de structures totalement différentes et qui ont le mérite en plus de conduire à un encombrement vraiment miniaturisé. Son seul point commun avec la valve du document FR-A-2 354 103 est d'utiliser des moyens de réglage magnétique.

La valve sous-cutanée selon l'invention pour le traitement de l'hydrocéphalie est à pression réglable de l'extérieur et destinée à être raccordée entre un cathéter ventriculaire et un cathéter de drainage, ladite valve comportant un corps à chambre cylindrique plate, un conduit d'amenée du liquide céphalorachidien dans ladite chambre, un clapet anti-retour (bille par exemple) placé à la sortie de ce conduit d'amenée, un ressort à lame poussant le clapet vers son siège et un organe mobile magnétique pouvant être commandé de l'extérieur en vue de modifier l'action du ressort sur le clapet, et est caractérisée en ce que l'orifice de sortie du canal d'amenée pourvu du clapet débouche dans la chambre sur la paroi cylindrique de celle-ci, en ce que le ressort à lame est arqué de manière à épouser ladite paroi cylindrique et en ce que la longueur de la partie active de la lame du ressort agissant sur le clapet est réglée par le positionnement de l'organe mobile le long de ladite paroi.

On connaît par le brevet américain 4 072 167 des valves dont le clapet (une bille) est poussé vers son siège par un ressort de torsion que l'on bande plus ou moins en modifiant angulairement la position de son extrémité fixe. Cette valve qui a d'ailleurs été prévue pour la régulation de circuits hydrauliques ne peut fonctionner que pour des hautes pressions, celles, par lesquelles, comme le reconnaît le brevet, les ressorts de compression hélicoïdaux deviennent inopérants.

La valve selon la présente invention ne peut utiliser que des ressorts à lame, seuls ceux-ci ayant la sensibilité requise.

D'autres caractéristiques de l'invention apparaîtront au cours de la description qui va suivre, faite en référence aux dessins annexés, donnés uniquement à titre d'exemple et sur lesquels :

la figure 1 est une vue en coupe d'une valve suivant l'invention, tarée pour une pression minimale du liquide céphalo-rachidien ; et

la figure 2 est une vue de la valve de la figure 1 tarée pour une pression maximale de fonctionnement.

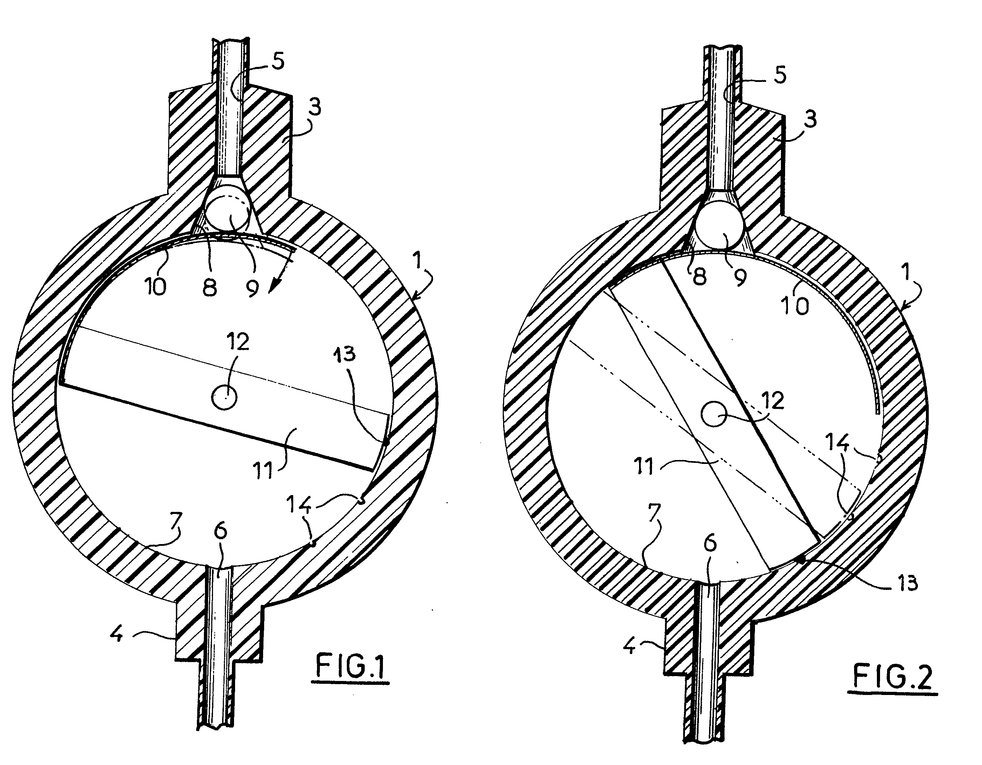

La valve représentée à la figure 1 comporte principalement un corps 1 de forme cylindrique aplatie, réalisé en une matière plastique appropriée.

Ce corps 1 comporte deux saillies 3 et 4 diamétralement opposées dans chacune desquelles sont respectivement ménagés un conduit 5 d'amenée du liquide céphalo-rachidien et un conduit 6 d'évacuation de ce liquide. Ces conduits 5 et 6 débouchent dans la paroi latérale d'une chambre 7 de forme cylindrique correspondante à celle du corps 1.

A l'extrémité du conduit 5 d'amenée du liquide intérieure au corps 1, est ménagé un siège 8 de forme tronconique pour une bille 9 formant clapet. Ce siège 8 peut être de position réglable.

La bille 9 est maintenue contre son siège par un ressort à lame arqué 10 qui s'étend le long d'une portion de la paroi latérale de la chambre 7. Ce ressort 10 occupe avantageusement un quart de la circonférence de la chambre 7.

Dans le mode de réalisation visible sur les figures, ce ressort arqué 10 est fixé par l'une de ses extrémités, donc en porte à faux à l'une des extrémités d'un barreau 11 en matériau magnétique monté à rotation dans la chambre 7 sur un axe 12 centré dans ladite chambre. La longueur du ressort est légèrement inférieure à la demi-circonférence de la chambre 7.

Sur son extrémité opposée au ressort 10, le barreau 11 porte une petite saillie 13 destinée à être engagée dans des évidements 14 ménagés dans la paroi latérale de la chambre afin d'assurer l'immobilisation du barreau 11 formant rotor, dans autant de positions qui correspondent à différentes pressions de fonctionnement de la valve.

Dans la position du rotor 11 représentée à la figure 1, on voit que le point de contact du ressort à lame 10 avec la bille 9 du clapet est éloigné du point d'ancrage de la lame 10 sur le rotor 11 de sorte que le moment résistant du ressort s'opposant à une poussée exercée par le liquide sur la bille 9 est faible.

Il en résulte que la bille peut quitter son siège pour une faible pression du liquide céphalo-rachidien amené par le conduit 5.

En revanche, dans la position du rotor 11 représentée à la figure 2, le point de contact du ressort à lame 10 avec la bille 9 du clapet, se trouve à proximité du point d'ancrage de la lame 10, de sorte que le ressort présente un moment résistant plus élevé correspondant à la pression maximale de fonctionnement de la valve.

On comprend aisément que seul un ressort à lame peut former ces deux types de pressions dont l'écart est néanmoins peu important alors que la variation angulaire du barreau porteur est très grande.

Si l'on fait un parallèle par exemple avec la valve du document FR-A-2 354 103, on constate que dans celle-ci la modification de la pression est inappréciable visuellement et nécessite l'utilisation d'un capteur inductif de déplacement.

Il est aisé d'imaginer, sans qu'il soit nécessaire de faire un nouveau dessin, que le ressort 10, aura lieu d'être mobile avec le barreau diamétral 11, soit fixé au corps par l'une des ses extrémités.

C'est alors la position de l'extrémité adjacente du barreau 11 sur la face intérieure de la lame qui détermine l'importance du moment résistant du ressort.

L'écoulement du liquide céphalo-rachidien entre le conduit d'amenée 5 et le conduit d'évacuation 6 se fait de part et d'autre du rotor 11 dont l'épaisseur est égale à la moitié environ de la hauteur de la chambre 7.

A chaque évidement 14 ménagé dans la paroi latérale de la chambre 7, correspond une position différente du point de contact du ressort 10 avec la bille 9 et par conséquent une pression de fonctionnement différente de la valve.

Bien entendu, l'ensemble représenté sur les dessins est enrobé dans une matière compatible avec les tissus telle qu'une matière à base d'élastomère de silicone (non représentée). Il est raccordé à un drain amont classique qui se termine par un cathéter ventriculaire et à un drain aval distal ou cathéter de drainage.

Le rotor 11 étant constitué par un barreau en matériau magnétique il est possible d'assurer les déplacements en rotation dudit barreau à l'aide d'un aimant dont l'action sur le barreau 11 s'exerce à travers la paroi de la valve et les tissus cutanés qui la recouvrent lorsqu'elle est mise en place.

Le neuro-chirurgien peut donc modifier la pression de fermeture de la valve à distance, sans aucun contact mécanique avec celle-ci, ce qui évite de procéder à une incision à cet effet. En outre, la valve qui vient d'être décrite est une valve programmable évitant l'emploi de plusieurs valves à pressions de fonctionnement différentes au cours de l'évolution de la maladie.

## Revendications

1. Valve sous-cutanée à pression réglable de l'extérieur pour le traitement de l'hydrocéphalie, destinée à être raccordée entre un cathéter ventriculaire et un cathéter de drainage, ladite valve comportant un corps (1) à chambre cylindrique plate (7), un conduit (5) d'amenée du liquide céphalo-rachidien dans ladite chambre (7), un clapet anti-retour (9) placé à la sortie de ce conduit d'amenée, un ressort à lame (10) poussant le clapet (9) vers son siège (8) et un organe mobile magnétique (11) pouvant être commandé de l'extérieur en vue de modifier l'action du ressort sur le clapet, caractérisée en ce que l'orifice de sortie du conduit d'amenée (5) pourvu du clapet (8/9) débouche dans la chambre (7) sur la paroi cylindrique de celle-ci, en ce que le ressort à lame (10) est arqué de manière à épouser ladite paroi cylindrique et en ce que la longueur de la partie active de la lame du ressort (10) agissant sur le clapet est déterminée par la position de l'organe mobile le long de la dite paroi cylindrique.

2. Valve selon la revendication 1, caractérisée en ce que l'une des extrémités du ressort est fixée à ladite paroi cylindrique, l'autre extrémité étant libre, l'organe mobile étant un bras diamétral ou rotor (11) dont l'une des extrémités s'applique sur ledit ressort.

3. Valve selon la revendication 1, caractérisée en ce que l'une des extrémités du ressort (10) est fixée à l'extrémité d'un bras diamétral ou rotor (11) en forme de barreau constituant ledit organe mobile, l'autre extrémité du ressort étant libre.

4. Valve selon l'une des revendications précédentes, caractérisée en ce que la lame élastique du ressort (10) occupe au moins un quart de la circonférence de la chambre (7).

5. Valve selon l'une des revendications 2 et 3, caractérisée en ce qu'elle comporte des moyens d'immobilisation du rotor et en ce que ces moyens résident en une saillie (13) coopérant avec des évidements (14) ménagés dans la paroi cylindrique de la chambre, et correspondant à des pressions prédéterminées de fonctionnement.

## Claims

1. Subcutaneous valve with pressure adjustable from outside, for the treatment of hydrocephalus, which is intended to be connected between a ventricular catheter and a drainage catheter, the said valve comprising a body (1) with a flat cylindrical chamber (7), a duct (5) delivering the cephalo-rachidian liquid into the said chamber (7), a non-return flap (9) located at the outlet of this delivery duct, a leaf spring (10) pushing the flap (9) towards its seat (8) and a moveable magnetic component (11) which can be controlled from outside in order to modify the action of the spring on the flap, characterised in that the outlet orifice of the delivery duct (5) provided with the flap (8/9) opens into the chamber (7) on the cylindrical wall of the latter, in that the leaf spring (10) is curved so as to match the said cylindrical wall, and in that the length of the active part of the leaf of the spring (10) acting on the flap is determined by the position of the moveable component along the said cylindrical wall.

2. Valve according to claim 1, characterised in that one of the ends of the spring is fastened to the said cylindrical wall, the other end being free, the moveable component being a diametral arm or rotor (11), one of the ends of which comes up against the said spring.

3. Valve according to claim 1, characterised in that one of the ends of the spring (10) is fastened to the end of a diametral arm or rotor (11) in the form of a bar constituting the said moveable component, the other end of the spring being free.

4. Valve according to one of the preceding claims, characterised in that the elastic leaf of the spring (10) occupies at least a quarter of the circumference of the chamber (7).

5. Valve according to one of claims 2 and 3, characterised in that it incorporates means for immobilising the rotor and in that these means consist of a projection (13) interacting with recesses (14) made in the cylindrical wall of the chamber and corresponding to predetermined operating pressures.

**Ansprüche**

1. Subkutanes, von außen druckregelbares Ventil für die Behandlung des Hydrozephalus, das dazu bestimmt ist, zwischen einem Ventrikulärkatheter und einem Drainagekatheter angeschlossen zu werden, wobei das Ventil einen Körper (1) mit einer flachen zylindrischen Kammer (7), eine Leitung (5) zur Zufuhr der zum verlängerten Rückenmark gehörigen Flüssigkeit in die genannte Kammer (7), eine am Ausgang dieser Zufuhrleitung angeordnete Rückschlagklappe (9), eine die Klappe (9) gegen ihren Sitz (8) drückende Blattfeder (10) und ein bewegliches magnetisches Organ (11) umfaßt, das von außen im Hinblick auf eine Änderung der Wirkung der Feder auf die Klappe steuerbar ist, dadurch gekennzeichnet, daß die Austrittsöffnung der mit der Klappe (8, 9) versehenen Zufuhrleitung (5) in die Kammer (7) an der zylindrischen Wand derselben mündet, daß die Blattfeder (10) derart gebogen ist, daß sie sich an die genannte zylindrische Wand anschmiegt, und daß die Länge des aktiven, auf die Klappe wirkenden Teiles der Blattfeder (10) durch die Stellung des beweglichen Organs längs der genannten zylindrischen Wand bestimmt ist.

2. Ventil nach Anspruch 1, dadurch gekennzeichnet, daß das eine der Enden der Feder an der zylindrischen Wand befestigt und das andere Ende frei ist, wobei das bewegliche Organ ein diametraler Arm oder Rotor (11) ist, dessen eines Ende an der genannten Feder anliegt.

3. Ventil nach Anspruch 1, dadurch gekennzeichnet, daß das eine der Enden der Feder (10) an dem Ende eines das bewegliche Organ bildenden diametralen Armes oder Rotors (11) in Stabform befestigt ist, wobei das andere Ende der Feder frei ist.

4. Ventil nach einem der vorhergehenden Ansprüche, dadurch gekennzeichnet, daß das elastische Blatt der Feder (10) mindestens ein Viertel des Umfanges der Kammer (7) einnimmt.

5. Ventil nach Anspruch 2 oder 3, dadurch gekennzeichnet, daß es Mittel zum Festhalten des Rotors umfaßt und daß diese Mittel aus einem Vorsprung (13) bestehen, der mit Aussparungen (14) zusammenwirkt, die in der Wand der zylindrischen Kammer ausgebildet sind und vorbestimmten Betriebsdrücken entsprechen.

FIG.1    FIG.2

0 060 369